# EUROPÄISCHE PATENTSCHRIFT

(11) **EP 1 415 964 B1**
(45) Veröffentlichungstag und Bekanntmachung des Hinweises auf die Patenterteilung: **13.02.2013**
(21) Anmeldenummer: 03024494.1
(22) Anmeldetag: 24.10.2003
(51) Int. Cl.: C07C 17/386, C07C 17/392, C07C 25/08

(54) **Verfahren zur Trennung von m- und p-Dichlorbenzol enthaltenden Gemischen**
Process for the separation of mixtures containing m- and p-dichlorobenzene
Procédé pour la séparation de mélanges contenant du m- et p-dichlorobenzène

(30) Priorität: 04.11.2002 DE 10251191
(43) Veröffentlichungstag der Anmeldung: 06.05.2004
(73) Patentinhaber: LANXESS Deutschland GmbH, 51369 Leverkusen (DE)
(72) Erfinder: Erdem, Gültekin, 21073 Hamburg (DE); Leckenbusch, Morris, 42277 Wuppertal (DE); Olf, Günter, Dr., 51373 Leverkusen (DE); Rinck, Kay-Jochen, Dr., 51465 Bergisch Gladbach (DE); Zühlke, Günter, 51503 Rösrath (DE)

(56) Entgegenhaltungen:
- EP-A- 0 451 720
- DE-A- 2 332 889
- US-A- 4 036 703
- DATABASE WPI Section Ch, Week 198728 Derwent Publications Ltd., London, GB; Class E15, AN 1987-194650 XP002271964 & JP 62 123135 A (SUMITOMO CHEM IND KK) 4. Juni 1987 (1987-06-04)

## Beschreibung

Bei dem herkömmlichen Herstellungsprozess von Dichlorbenzolen über die Chlorierung von Benzol, fallen neben den drei isomeren Dichlorbenzolen (DCB), auch Chlorbenzol und höher chlorierte Benzole (z.B. Trichlorbenzole) an. Während sich das Monochlorbenzol und die höher chlorierten Benzole leicht von den Dichlorbenzolen destillativ abtrennen lassen, gehört die Gewinnung von reinem m-Dichlorbenzol und reinem p-Dichlorbenzol zu den schwierigsten Trennaufgaben der aromatischen Zwischenproduktchemie. Denn die Siedepunkte zwischen m- und p-DCB liegen weniger als 1°C auseinander, so dass die destillative Trennung in hoher Reinheit durch fraktionierte Rektifikation praktisch unmöglich ist.

Die Erfindung betrifft ein Verfahren zur Trennung von m-/p-Dichlorbenzol durch extraktive Rektifikation mit Extraktionsmitteln und Abtrennung dieser Extraktionsmittel. Durch das erfindungsgemäße Verfahren können m-Dichlorbenzol und p-Dichlorbenzol, aus Gemischen die m-Dichlorbenzol und p-Dichlorbenzol enthalten, in hoher Reinheit erhalten werden. Reine Dichlorbenzole sind wichtige Zwischenprodukte für Farbstoffe, Riechstoffe und Pharmazeutika.

Aufgrund der schwierigen Trennung von m-Dichlorbenzol und p-Dichlorbenzol gibt es auch Ansätze, über komplizierte chemische Umwege, die Isomere in reiner Form zu erhalten. So wird beispielsweise in JP 53044528 das m-Dichlorbenzol und das o-Dichlorbenzol in einer Dichlorbenzolmischung mit Hilfe von Schwefelsäure selektiv sulfoniert. Nach Abtrennung des nicht-reagierten p-Dichlorbenzols werden m-Dichorbenzol und o-Dichlorbenzol durch Desulfonierung bei höherer Temperatur erhalten. In US-PS 3 170 961 wird der Umweg über Brom-Isomere vorgeschlagen. In diesem Verfahren werden die Dichlorbenzole bromiert. Die Brom-Isomere können durch Destillation getrennt werden, um dann durch Abspaltung von Brom reine Dichlorbenzole zu erhalten. Diese Verfahren sind jedoch schwierig und teuer.

Mit Hilfe der Schmelzkristallisation, wie sie für die Trennung von vielen aromatischen Isomeren mit Erfolg angewendet wird, ist die Auftrennung von Gemischen aus m-Dichlorbenzol und p-Dichlorbenzol in reines m-Dichlorbenzol und reines p-Dichlorbenzol nicht möglich, da das binäre System bei einem Gehalt von 88 Gew.-% m-Dichlorbenzol ein Eutektikum zeigt. Mit dieser Trennmethode kann nur eines der beiden Isomere in reiner Form erhalten werden. Zudem werden für die Isolierung von m-Dichlorbenzol tiefe Temperaturen (ca. -30°C) benötigt, was den Prozess unwirtschaftlich macht.

In der Literatur werden weiterhin Verfahren zur Trennung von m-Dichlorbenzol und p-Dichlorbenzol beschrieben, die auf der unterschiedlichen Adsorption an Zeolithen beruhen. So werden zwar in z.B. JP 11 158 093 Trennfaktoren zwischen m-DCB und p-DCB genannt, die durchaus technisch zu reinen Isomeren führen, diese Verfahren haben jedoch den Nachteil, dass die Regeneration des Zeolithen aufwendig ist und hohe Mengen an Lösemitteln im Kreis gefahren werden müssen.

Eine besonders günstige Trenntechnik ist die Extraktivrektifikation. Dazu wird dem zu trennenden Gemisch ein Extraktionsmittel zugegeben, welches über selektive Wechselwirkungen das Dampf-Flüssig-Phasengleichgewicht in der Art beeinflusst, dass Trennfaktoren von ungleich eins erhalten werden. In der Schrift JP 58 174 333 werden dazu Anilinderivate genannt. Danach werden Trennfaktoren im Bereich von 1.08 bis 1.16 erhalten. In dieser Substanzklasse sind einige Stoffe dabei, die einen ungünstigen Siedepunkt zeigen und zudem sind einige Stoffe technisch schwer herzustellen und somit teuer. In JP 54 160 322 werden die Stoffe Sulfolan (Trennfaktor 1,15), Decanol (Trennfaktor 1,1) und die drei Isomeren des Kresols (Trennfaktoren 1,07 bis 1,14) angegeben. Sulfolan ist wegen seines hohen Siedepunktes und seiner chemischen Unbeständigkeit bei höheren Temperaturen nicht gut geeignet für die Trennung der DCB-Isomere. In einer Dissertation (Unverdorben, L., Universität Erlangen-Nürnberg, 1992) wurden die Trennfaktoren von Alkylencarbonaten (Ethylencarbonat, Propylencarbonat) bestimmt. Danach werden Trennfaktoren von 1,13 bis 1,14 erhalten.

In DE 23 32 889A und EP 0 451 720A wird die Trennung von m- und p-Dichlorbenzol unter Verwendung von Extraktionsmitteln in einer Rektifikation beschrieben, wobei als Extraktionsmittel Hexamethylphosphortriamid, Dimethylsulfoxid, Dibutylsulfoxid und/oder N-Methylpyrrolidon bzw. Alkylencarbonate eingesetzt werden.

In US 4,036,703A wird die Trennung von chlorierten C₁-C₃-Alkanen unter Verwendung einer Vielzahl von strukturell unterschiedlichsten Extraktionsmitteln, wie z.B. Tri-n-butylphosphat, beschrieben.

Aus DATABASE WPI Section CH, Week 198728 Thomson Scientific, London, GB; Class E15, AN 1987-194650 XP002271964 & JP 62 123 135A (SUMITOMO CHEM IND KK) 4. Juni 1987 (1987-06-04) ist die Trennung von Benzol-Cyclohexen-Cyclohexan-Gemischen, sowie Toluol-Methylcyclohexen-Methylencyclohexan-Gemischen unter Verwendung von Phosphorsäureestern bekannt.

Die oben genannten Stoffe zeigen jedoch einige Nachteile. Trennfaktoren von < 1,15 erfordern eine sehr hohe Trennleistung. Einige der bekannten Stoffe sind mehr oder weniger toxisch. Bei einigen Stoffen liegen die Siedepunkte ungünstig, so dass die Trennung zwischen Extraktionsmittel und p-DCB aufwendig ist. Zudem zeigen einige Stoffe Zersetzung, so dass sie nicht mehrfach eingesetzt werden können.

Es bestand die Aufgabe, ein Verfahren auf Basis der Extraktivrektifikation zu entwickeln, mit dem m-DCB und p-DCB getrennt werden können, aus Gemischen die m-DCB und p-DCB enthalten. Gegenüber dem Stand der Technik sollen die dazu notwendigen Extraktionsmittel Trennfaktoren (m-DCB/p-DCB) von deutlich ungleich eins erzeugen, damit der Trennaufwand reduziert wird. Der Zusatzstoff sollte insbesondere höher sieden als das zu trennende Isomerenpaar m-DCB und p-DCB. Weiterhin soll die Siedepunktsdifferenz bei 1 bar (1 atm) zwischen dem Extraktionsmittel und p-DCB möglichst mindestens 20°C, bester mindestens 35°C betragen um eine einfache destillative Abtrennung des Extraktionsmittels vom p-DCB zu ermöglichen.

Die Extraktionsmittel sollen zudem toxikologisch und ökologisch unbedenklich sein. Weiterhin sollen aus Kostengründen entweder Extraktionsmittel mit hoher Langzeitstabilität oder großtechnisch verfügbare preiswerte Extraktionsmittel eingesetzt werden.

Diese Aufgabe wird erfindungsgemäß dadurch gelöst, dass ausgewählte Phosphorsäureester, z.B. Triethylphosphat und Phosphinoxide, z.B. Tri-n-propylphosphinoxid oder Tri-n-butylphosphinoxid oder deren Gemische im Prozess als Extraktionsmittel eingesetzt werden.

Gegenstand der Erfindung ist ein Verfahren zur Trennung von Chlorbenzol-Gemischen enthaltend m- und p-Dichlorbenzol mittels extraktiver Rektifikation unter Verwendung eines Extraktionsmittels, Trennung der Komponenten in eine m-Dichlorbenzol und p-Dichlorbenzol enthaltende Fraktion und abschließende Abtrennung des Extraktionsmittels von einer der erhaltenen Fraktionen, dadurch gekennzeichnet, dass als Extraktionsmittel ein Phosphorsäureester der allgemeinen Formel (I) 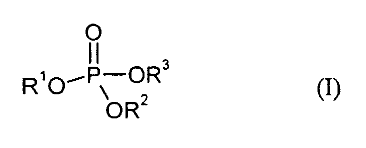 wobei R¹, R² und R³ gleich oder verschieden sind und für einen aliphatischen oder cycloaliphatischen Alkyl- oder Alkylenrest stehen und R¹, R² und R³ zusammen mindestens 3 und maximal 12 C-Atome enthalten, oder eine Mischung verschiedener derartiger Phosphorsäureester oder ein Phosphinoxid der allgemeinen Formel (II) 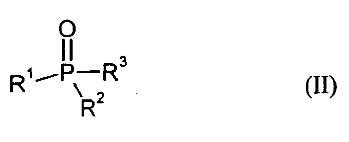 wobei R¹, R² und R³ gleich oder verschieden sind und für einen aliphatischen oder cycloaliphatischen Alkyl- oder Alkylenrest oder Wasserstoff stehen und hierbei aber R¹, R² und R³ zusammen mindestens 3 und maximal 12 C-Atome enthaltenen, oder eine Mischung verschiedener derartiger Phosphinoxide oder eine Mischung der genannten Phosphorsäureester und Phosphinoxide verwendet wird.

Die oben genannten Alkanreste und Alkenreste können bevorzugt unverzweigt, verzweigt, cyclisch, gesättigt und ungesättigt sein.

Bevorzugt stehen in der Formel (I) oder (II) für das Extraktionsmittel R¹, R² und R³ gleich oder verschieden für einen Rest aus der Reihe: Methyl, Ethyl, n-Propyl, Isopropyl, n-Butyl, tert-Butyl, n-Pentyl, sec.-Butyl.

Besonders bevorzugt wird als Extraktionsmittel Triethylphosphat, Tripropylphosphinoxid oder Tributylphosphinoxid allein oder in Mischung verwendet.

In einem bevorzugten Verfahren wird die Trennung in einer Rektifikationskolonne durchgeführt, wobei der Druck am Kolonnenkopf im Bereich von 5 bis 100 hPa, und die Druckdifferenz zwischen Kolonnensumpf und Kolonnenkopf 0 bis 100 hPa beträgt und gegebenenfalls die Anzahl theoretischer Trennstufen 20 bis 200 beträgt.

Besonders bevorzugt beträgt der Druck am Kolonnenkopf 5 bis 30 hPa, und die Druckdifferenz zwischen Kolonnensumpf und Kolonnenkopf 0 bis 20 hPa und gegebenenfalls die Anzahl theoretischer Trennstufen 60 bis 120.

Das Gewichtsverhältnis von Rücklauf zu Destillat beträgt in einer bevorzugten Ausführung des Verfahrens 1 : 1 bis 20:1, besonders bevorzugt 3:1 1 bis 8:1.

Das Gewichtsverhältnis von Zulauf des Extraktionsmittels zu Zulauf des m-/p-Dichlorbenzolgemisches beträgt in einer weiteren Form des Verfahrens insbesondere 2:1 bis 40:1, insbesondere bevorzugt 6:1 bis 12:1.

Das Verfahren ist durch die Verwendung von Phosphorsäureestern und Phosphinoxiden der Formeln (I) oder (II) als Extraktionsmittel für die Extraktivrektifikation gekennzeichnet.

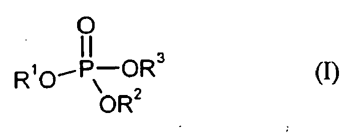

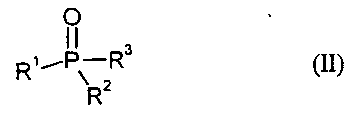

Hierin weisen die Reste R¹, R² und R³ die oben angegebene Bedeutung auf.

Mit der Erfindung ist es möglich, aus Mischungen die m-DCB und p-DCB enthalten, durch Extraktivrektifikation Fraktionen mit m-DCB und Fraktionen mit p-DCB zu erhalten, die praktisch das andere Isomer nicht mehr enthalten. Das Einsatzgemisch kann mehr oder viel mehr m-DCB als p-DCB enthalten. Das Einsatzgemisch kann auch mehr oder viel mehr p-DCB als m-DCB enthalten. Das Einsatzgemisch kann auch die beiden Isomere in gleicher Konzentration enthalten. Für den gesamten Konzentrationsbereich können hohe Reinheiten von nahezu 100 % erreicht werden. Das Isomerengemisch kann neben diesen beiden DCB-Isomeren noch weitere chlorierte Benzole, wie z.B. Monochlorbenzol, o-Dichlorbenzol und höher chlorierte Aromaten enthalten. Weiterhin können Stoffe enthalten sein, die üblicherweise bei der Chlorierung von Benzol vorliegen oder bei der Reaktion gebildet werden, wie z.B. chlorierte Nitrochlorbenzole, Benzol, Chlorwasserstoff und Katalysator.

Zum Beweis der Wirksamkeit der erfindungsgemäß verwendeten Extraktionsmittel sind in nachfolgender Tabelle die Trennfaktoren zusammengestellt. Mit Ausnahme der Daten zum Tributylphosphinoxid wurden diese Werte bei 120°C nach der Headspacemethode gemessen, die bereits früher in Verfahrenstechnik 8 (1974) Nr.12, S. 343-347 erläutert wurde. Die Messungen wurden bei einer Konzentration in der Flüssigkeit von 10 Mol-% m-DCB, 10 Mol-% p-DCB und 80 Mol-% Extraktionsmittel ausgeführt. Die Daten zum Tributylphosphinoxid wurden mit einer dynamischen Gleichgewichtsapparatur bei 80 hPa gemessen und mit den Mitteln der klassischen Thermodynamik auf 120°C und 10 Mol-% m-DCB, 10 Mol-% p-DCB und 80 Mol-% Tri-n-butylphosphinoxid umgerechnet. Zum Vergleich in der Wirksamkeit dieser Extraktionsmittel sind auch die entsprechenden Messwerte für Sulfolan und Propylencarbonat in die Tabelle aufgenommen worden. Der Wert zu Propylencarbonat ist ein Messwert von Unverdorben, L., Dissertation, Universität Erlangen-Nürnberg, 1992.

| Extraktionsmittel | Trennfaktor |
|---|---|
| Triethylphosphat | 1,23 |
| Tri-n-propylphosphinoxid | 1,21 |
| Tri-n-butylphosphinoxid | 1,17 |
| Sulfolan | 1,14 |
| Propylencarbonat | 1,14 |

Die Prüfungen ergaben, dass die Phosphorsäureester und die Phosphinoxide im Vergleich zu den anderen Stoffen sehr gute Trennfaktoren zeigen. Langzeitstabilitätsuntersuchungen zu den Phosphorsäureestern/Dichlorbenzol-Gemischen zeigen, dass auch nach Wochen nur sehr geringe Anteile an Ester wegreagiert ist. Die Phosphinoxide zeigen ebenfalls eine hohe thermische Stabilität. Weiterhin zeigen beide Substanzklassen unter den relevanten Bedingungen keine Reaktionen mit chlorierten Aromaten. Phosphorsäureester und Phosphinoxide sind daher und aufgrund ihrer überwiegend erst im Rahmen dieser Erfindung ermittelten positiven Stoffeigenschaften und nicht zuletzt auch wegen leichter Abtrennbarkeit von den Dichlorbenzolen als Extraktionsmittel für eine Extraktivrektifikation von m-/p-DCB-Gemischen hervorragend geeignet.

Im Allgemeinen weist das in die Extraktionskolonne eingespeiste, zu trennende Gemisch eine Temperatur auf, die in Abhängigkeit vom gewählten Kolonnendruck zwischen 20 und 80°C und vorzugsweise zwischen 40 und 60°C liegt. Die Zulauftemperatur des Extraktionsmittels liegt zweckmäßigerweise zwischen 50 und 70°C. Die Sumpftemperatur in der Extraktivrektifikationskolonne sollte zweckmäßigerweise 180°C vorzugsweise 130°C nicht überschreiten.

Das erfindungsgemäße Verfahren kann auch dazu verwendet werden, um aus einer Mischung aus m-DCB und p-DCB reines p-DCB zu gewinnen oder um aus einer Mischung aus m-DCB und p-DCB sowohl reines m-DCB als auch reines p-DCB zu gewinnen.

Das Verfahren der Extraktivrektifikation ermöglicht insbesondere auch Anwendungen zur Trennung von m-DCB und p-DCB, in denen die Extraktivrektifikation mit einer Schmelzkristallisation oder einer Chromatographie kombiniert wird, um sehr hohe Reinheiten zu erhalten.

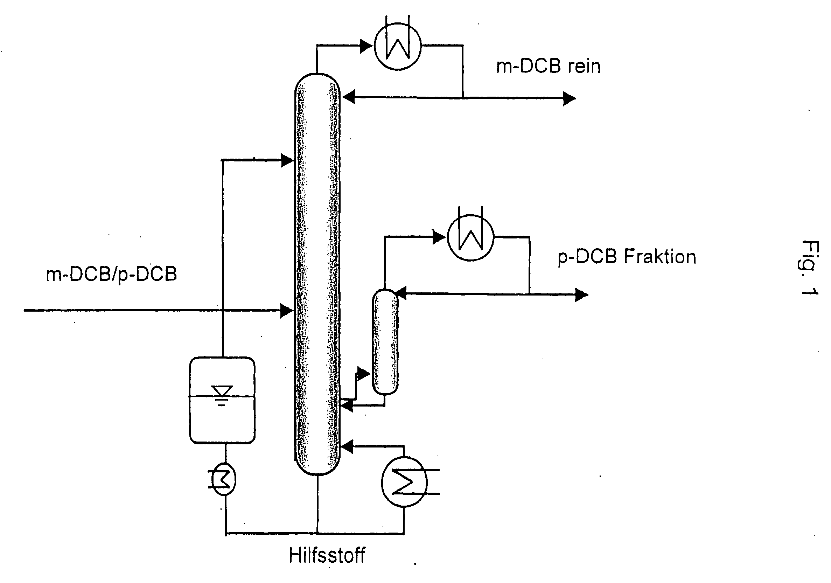

Das Verfahren ermöglicht insbesondere auch Anwendungen zur Trennung von m-DCB und p-DCB, bei der sowohl die Extraktivrektifikation von m-DCB und p-DCB als auch die gemeinsame Abtrennung des p-DCB und des restlichen m-DCB vom Extraktionsmittel in einer einzigen Kolonne mit dampfförmiger Seitenstromentnahme erfolgt. Die Kolonne weist in diesem Fall bevorzugt einen an die dampfförmige Seitenstromentnahme angeschlossenen mehrstufigen Kolonnenverstärkungsabschnitt (Seitenstromkolonne) mit Kopfkondensator und Rücklaufteiler auf, in dem das p-DCB und das restliche m-DCB vom Extraktionsmittel abgetrennt wird. Im Kopf der Kolonne wird das reine m-DCB und im Sumpf das reine Extraktionsmittel gewonnen, welches wieder zurück in die Kolonne geführt wird (vgl. Fig. 1). Die Qualität des Kopfproduktes wird hierbei mittels geeigneter Methoden z.B. über eine Produktanalyse oder eine Online-Analytik sichergestellt, welche auf das Rücklaufverhältnis in der Kolonne entsprechend Einfluss nimmt. Die Seitenstromentnahmemenge wird z.B. über eine Temperaturmessung an einer entsprechend sensiblen Stelle in der mit konstantem Rücklaufverhältnis betriebenen Seitenstromkolonne geregelt, welche den Öffnungsgrad eines Ventils in der Kondensatleitung hinter dem Kopfkondensator der Seitenstromkolonne reguliert. Aufgrund der damit verbundenen Einflussnahme auf den Flüssigkeitsstand und damit auf die wirksame Fläche in diesem Kondensator erfolgt die selbsttätige Regulierung der TEP-Konzentration im entnommenen Seitenstrom.

Die Trennung von m- und p-Dichlorbenzol sowie die Rückgewinnung des Extraktionsmittels wird bevorzugt in einer Rektifikationskolonne durchgeführt, wobei zur Rückgewinnung des Extraktionsmittels an die Rektifikationskolonne über eine dampfförmige Seitenstromentnahme eine Seitenstromkolonne angeschlossen ist.

Das Verfahren ermöglicht insbesondere auch Anwendungen, bei dem das Extraktionsmittel vom p-Dichlorbenzol durch Destillation oder durch Extraktion oder durch Kristallisation abgetrennt wird.

Besonders vorteilhaft ist eine Variante des Verfahrens, in der der Extraktiv-Rektifikation eine Schmelzkristallisation nachgeschaltet ist um mit reduziertem Energieaufwand das gewünschte Isomere insbesondere p-Dichlorbenzol, in sehr hoher Reinheit (>>99 Gew.-%) zu erhalten.

### Beispiel

Eine Laborkolonne mit 30 mm Innendurchmesser, mit einem innen verspiegelten, außen beheizten Vakuummantel, die mit 3 mm x 3 mm (Maschenweite) Maschendrahtringen gepackt ist und eine trennwirksame Höhe von 2,5 m aufweist, wird in 1,0 m Höhe mit dem zu trennenden Gemisch bestehend aus 75 Gew.-% m-DCB und 25 % Gew.-p-DCB mit einer Temperatur von 50°C und in 2,0 m Höhe mit dem Extraktionsmittel Triethylphosphat mit einer Temperatur von 60°C gespeist.

Der Druck am Kolonnenkopf beträgt 10 hPa und im Kolonnensumpf 30 hPa. Das Extraktionsmittel verlässt den Kolonnensumpf flüssig siedend. Im Sumpfstrom sind weiterhin weniger als die Hälfte des eingesetzten m-DCB und mehr als 99,5 % des eingesetzten p-DCB enthalten. Am Kopf der Kolonne wird das im Kondensator kondensierte m-DCB abgezogen, welches weniger als 1 Gew.-% p-DCB enthält. Die abzuziehende Destillatmenge aus hochreinem m-DCB wird auf 25 g/h und das Rücklaufverhältnis (Rückflussmenge/Destillat) auf 5 eingestellt, während 60 g/h zu trennendes Gemisch und 500 g/h Triethylphosphat in die Kolonne eingespeist werden.

In einer zweiten Kolonne, die ebenfalls 30 mm Innendurchmesser, einen innen verspiegelten, außen beheizten Vakuummantel aufweist und mit 3 mm x 3 mm Maschendrahtringen gepackt ist, werden das im Sumpf der Extraktivrektifikationskolonne anfallende p-DCB und das restliche m-DCB gemeinsam vom Extraktionsmittel abgetrennt. Die trennwirksame Höhe dieser zweiten Kolonne beträgt ebenfalls 2,5 m. Die Kolonne wird in 1,5 m Höhe mit dem Zulaufgemisch aus p-DCB, restlichem m-DCB und Extraktionsmittel gespeist, welches zuvor in einem Kühler auf 65 °C abgekühlt wurde.

In der zweiten Kolonne beträgt der Kopfdruck ebenfalls 10 hPa und der Sumpfdruck ebenfalls 30 hPa. Das Extraktionsmittel verlässt den Kolonnensumpf flüssig siedend in reinem Zustand, während am Kopf der Kolonne das im Kondensator kondensierte Gemisch aus p-DCB und restlichem m-DCB abgezogen wird. Das Rücklaufverhältnis wird in dieser Kolonne auf 2 eingestellt. Das im Sumpf anfallende reine Extraktionsmittel wird in einem nachfolgenden Kühler auf 65°C abgekühlt und in ein 5 Liter fassendes Puffergefäß gefahren.

Das in die Extraktivrektifikationskolonne einströmende Extraktionsmittel wird aus diesem Puffergefäß gespeist, so dass ein geschlossener Extraktionsmittelkreislauf vorliegt.

Mit dem beschriebenen Experiment wurde ein Gemisch aus 75 Gew.-% m-DCB und 25 Gew.-% p-DCB in ein Destillat mit >99 Gew.-% m-DCB und in ein mit p-DCB angereicherten Ablauf aufgetrennt.

## Patentansprüche

1. Verfahren zur Trennung von Dichlorbenzol-Gemischen enthaltend m- und p-Dichlorbenzol mittels extraktiver Rektifikation unter Verwendung eines Extraktionsmittels, Trennung der Komponenten in eine m-Dichlorbenzol und p-Dichlorbenzol enthaltende Fraktion und abschließende Abtrennung des Extraktionsmittels von einer der erhaltenen Fraktionen, **dadurch gekennzeichnet, dass** als Extraktionsmittel ein Phosphorsäureester der allgemeinen Formel (I) 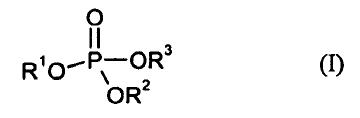 wobei R¹, R² und R³ gleich oder verschieden sind und für einen aliphatischen oder cycloaliphatischen Alkyl- oder Alkylenrest stehen und R¹, R² und R³ zusammen mindestens 3 und maximal 12 C-Atome enthalten, oder eine Mischung verschiedener derartiger Phosphorsäureester oder ein Phosphinoxid der allgemeinen Formel (II) 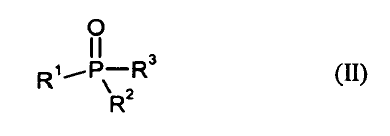 wobei R¹, R² und R³ gleich oder verschieden sind und für einen aliphatischen oder cycloaliphatischen Alkyl- oder Alkylenrest oder Wasserstoff stehen und hierbei aber R¹, R² und R³ zusammen mindestens 3 und maximal 12 C-Atome enthaltenen, oder eine Mischung verschiedener derartiger Phosphinoxide oder eine Mischung der genannten Phosphorsäureester und Phosphinoxide verwendet wird.

2. Verfahren nach Anspruch 1, **dadurch gekennzeichnet, dass** in der Formel (I) oder (II) für das Extraktionsmittel R¹, R² und R³ gleich oder verschieden für einen Rest aus der Reihe: Methyl, Ethyl, n-Propyl, Isopropyl, n-Butyl, tert-Butyl, n-Pentyl, sec.-Butyl stehen.

3. Verfahren nach Anspruch 1, **dadurch gekennzeichnet, dass** als Extraktionsmittel Triethylphosphat, Tripropylphosphinoxid oder Tributylphosphinoxid allein oder in Mischung verwendet wird.

4. Verfahren nach einem der Ansprüche 1 bis 3, **dadurch gekennzeichnet, dass** die Trennung in einer Rektifikationskolonne durchgeführt wird, wobei der Druck am Kolonnenkopf im Bereich von 5 bis 100 hPa, und die Druckdifferenz zwischen Kolonnensumpf und Kolonnenkopf 0 bis 100 hPa beträgt und gegebenenfalls die Anzahl theoretischer Trennstufen 20 bis 200 beträgt.

5. Verfahren nach Anspruch 4, **dadurch gekennzeichnet, dass** der Druck am Kolonnenkopf 5 bis 30 hPa, und die Druckdifferenz zwischen Kolonnensumpf und Kolonnenkopf 0 bis 20 hPa beträgt und gegebenenfalls die Anzahl theoretischer Trennstufen 60 bis 120 beträgt.

6. Verfahren nach einem der Ansprüche 1 bis 5, **dadurch gekennzeichnet, dass** das Gewichtsverhältnis des Massenstroms von Rücklauf zu Destillat 1:1 bis 20:1, insbesondere 3:1 bis 8:1 beträgt.

7. Verfahren nach einem der Ansprüche 1 bis 6, **dadurch gekennzeichnet, dass** das Gewichtsverhältnis des Massenstroms von Zulauf des Extraktionsmittels zu Zulauf des m-/p-Dichlorbenzolgemisches 2:1 bis 40:1, insbesondere 6:1 bis 12:1 beträgt.

8. Verfahren nach einem der Ansprüche 1 bis 7, **dadurch gekennzeichnet, dass** die Trennung von m- und p-Dichlorbenzol sowie die Rückgewinnung des Extraktionsmittels in einer Rektifikationskolonne durchgeführt wird, wobei zur Rückgewinnung des Extraktionsmittels an die Rektifikationskolonne über eine dampfförmige Seitenstromentnahme eine Seitenstromkolonne angeschlossen ist.

9. Verfahren nach einem der Ansprüche 1 bis 8, **dadurch gekennzeichnet, dass** der Extraktiv-Rektifikation eine Schmelzkristallisation zur Feinreinigung des gewünschten Isomeren insbesondere p-DCB nachgeschaltet ist.

## Claims

1. Process for the separation of dichlorobenzene mixtures containing m- and p-dichlorobenzene by means of extractive rectification using an extracting agent, separation of the components into an m-dichlorobenzene- and p-dichlorobenzene-containing fraction and final separation of the extracting agent from one of the fractions obtained, **characterized in that** the extracting agent used is a phosphoric ester of the general formula (I) 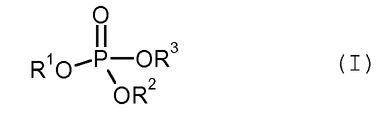 in which R¹, R² and R³ are identical or different and represent an aliphatic or cycloaliphatic alkyl or alkylene radical and R¹, R² and R³ together contain at least 3 and not more than 12 C atoms, or a mixture of different phosphoric esters of this type or a phosphine oxide of the general formula (II) 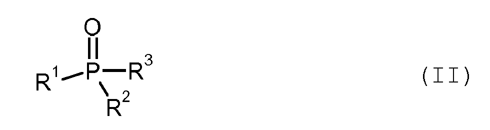 in which R¹, R² and R³ are identical or different and represent an aliphatic or cycloaliphatic alkyl or alkylene radical or hydrogen, but here R¹, R² and R³ together contain at least 3 and not more than 12 C atoms, or a mixture of different phosphine oxides of this type or a mixture of said phosphoric esters and phosphine oxides.

2. Process according to Claim 1, **characterized in that**, in the formula (I) or (II) for the extracting agent, R¹, R² and R³ are identical or different and represent a radical from the series: methyl, ethyl, n-propyl, isopropyl, n-butyl, tert-butyl, n-pentyl, sec-butyl.

3. Process according to Claim 1, **characterized in that** the extracting agent used is triethyl phosphate, tripropylphosphine oxide or tributylphosphine oxide alone or as a mixture.

4. Process according to any of Claims 1 to 3, **characterized in that** the separation is carried out in a rectification column, the pressure at the top of the column being in the range of 5 to 100 hPa and the pressure difference between the bottom of the column and the top of the column being 0 to 100 hPa and optionally the number of theoretical plates being 20 to 200.

5. Process according to Claim 4, **characterized in that** the pressure at the top of the column is 5 to 30 hPa and the pressure difference between the bottom of the column and the top of the column is 0 to 20 hPa and optionally the number of theoretical plates is 60 to 120.

6. Process according to any of Claims 1 to 5, **characterized in that** the weight ratio of the mass flow of reflux to distillate is 1:1 to 20:1, in particular 3:1 to 8:1.

7. Process according to any of Claims 1 to 6, **characterized in that** the weight ratio of the mass flow of feed of the extracting agent to feed of the m-/p-dichlorobenzene mixture is 2:1 to 40:1, in particular 6:1 to 12:1.

8. Process according to any of Claims 1 to 7, **characterized in that** the separation of m- and p-dichlorobenzene and the recovery of the extracting agent is carried out in a rectification column, a side-stream column being connected to the rectification column via a vapour side-stream take-off for recovery of the extracting agent.

9. Process according to any of Claims 1 to 8, **characterized in that** a melt crystallization for fine purification of the desired isomer, in particular p-DCB, is provided downstream of the extractive rectification.

## Revendications

1. Procédé de séparation de mélanges de dichlorobenzène contenant du m- et p-dichlorobenzène par rectification extractive utilisant un agent d'extraction, séparation des composants en une fraction contenant du m-dichlorobenzène et du p-dichlorobenzène et séparation finale de l'agent d'extraction d'une des fractions obtenues, **caractérisé en ce qu'**un ester de l'acide phosphorique de formule générale (I) 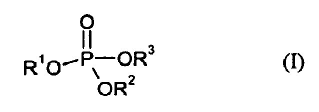 dans laquelle R¹, R² et R³ sont identiques ou différents et représentent un radical alkyle ou alkylène aliphatique ou cycloaliphatique et R¹, R² et R³ contiennent ensemble au moins 3 et au plus 12 atomes C, ou un mélange de tels esters de l'acide phosphorique différents ou un oxyde de phosphine de formule générale (II) 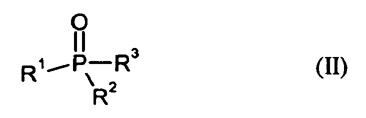 dans laquelle R¹, R² et R³ sont identiques ou différents et représentent un radical alkyle ou alkylène aliphatique ou cycloaliphatique ou l'hydrogène, et R¹, R² et R³ contiennent ensemble au moins 3 et au plus 12 atomes C, ou un mélange de tels oxydes de phosphine différents, ou un mélange des esters de l'acide phosphorique et des oxydes de phosphine cités, est utilisé en tant qu'agent d'extraction.

2. Procédé selon la revendication 1, **caractérisé en ce que**, dans la formule (I) ou (II) pour l'agent d'extraction, R¹, R² et R³ représentent de manière identique ou différente un radical de la série : méthyle, éthyle, n-propyle, isopropyle, n-butyle, tert-butyle, n-pentyle, sec.-butyle.

3. Procédé selon la revendication 1, **caractérisé en ce que** le phosphate de triéthyle, l'oxyde de tripropylphosphine ou l'oxyde de tributylphosphine est utilisé seul ou en mélange en tant qu'agent d'extraction.

4. Procédé selon l'une quelconque des revendications 1 à 3, **caractérisé en ce que** la séparation est réalisée dans une colonne de rectification, la pression à la tête de la colonne étant dans la plage allant de 5 à 100 hPa et la différence de pression entre le fond de la colonne et la tête de la colonne étant de 0 à 100 hPa, et le nombre d'étapes de séparation théoriques étant éventuellement de 20 à 200.

5. Procédé selon la revendication 4, **caractérisé en ce que** la pression à la tête de la colonne est de 5 à 30 hPa et la différence de pression entre le fond de la colonne et la tête de la colonne est de 0 à 20 hPa, et le nombre d'étapes de séparation théoriques est éventuellement de 60 à 120.

6. Procédé selon l'une quelconque des revendications 1 à 5, **caractérisé en ce que** le rapport en poids entre le débit massique de reflux et de distillat est de 1:1 à 20:1, notamment de 3:1 à 8:1.

7. Procédé selon l'une quelconque des revendications 1 à 6, **caractérisé en ce que** le rapport en poids entre le débit massique de l'alimentation de l'agent d'extraction et de l'alimentation du mélange m-/p-dichlorobenzène est de 2:1 à 40:1, notamment de 6:1 à 12:1.

8. Procédé selon l'une quelconque des revendications 1 à 7, **caractérisé en ce que** la séparation du m- et p-dichlorobenzène et la récupération de l'agent d'extraction sont réalisées dans une colonne de rectification, une colonne à courant latéral étant connectée à la colonne de rectification par un soutirage de courant latéral gazeux pour la récupération de l'agent d'extraction.

9. Procédé selon l'une quelconque des revendications 1 à 8, **caractérisé en ce qu'**une cristallisation par fusion pour la purification fine de l'isomère souhaité, notamment le p-DCB, est connectée en aval de la rectification extractive.
